(19) Europäisches Patentamt
European Patent Office
Office européen des brevets

(11) Veröffentlichungsnummer : **0 257 334 B1**

(12) # EUROPÄISCHE PATENTSCHRIFT

(45) Veröffentlichungstag der Patentschrift : **02.01.91 Patentblatt 91/01**

(21) Anmeldenummer : **87111024.3**

(22) Anmeldetag : **30.07.87**

(51) Int. Cl.[5] : **C07D 487/04**, C07D 487/10, C07D 498/04, C07D 498/10, C07D 513/04, C07D 513/10, A61K 31/41, A61K 31/415, A61K 31/42, A61K 31/425, // (C07D487/04, 249:00, 209:00),(C07D498/04, 263:00, 249:00),(C07D487/04, 249:00, 235:00)

(54) **Tricyclische Benzotriazole, Verfahren zu ihrer Herstellung sowie Arzneimittel.**

Die Akte enthält technische Angaben, die nach dem Eingang der Anmeldung eingereicht wurden und die nicht in dieser Patentschrift enthalten sind.

(30) Priorität : **07.08.86 DE 3626664**

(43) Veröffentlichungstag der Anmeldung : **02.03.88 Patentblatt 88/09**

(45) Bekanntmachung des Hinweises auf die Patenterteilung : **02.01.91 Patentblatt 91/01**

(84) Benannte Vertragsstaaten : **AT BE CH DE ES FR GB GR IT LI LU NL SE**

(56) Entgegenhaltungen :
**EP-A- 0 161 632**
**EP-A- 0 186 010**
**DE-A- 3 501 497**

(56) Entgegenhaltungen :
**CHEMICAL ABSTRACTS, Band 73, no. 21, 23. November 1970, Columbus, Ohio, USA; TERENT'EV, A. P. et al. "Introduction of substituents into the benzene ring of indole. XII. Structure of heterocyclic systems based on 5,6-substituted indolines." Seiten 368, 369, Zusammenfassung Nr. 109 741a**

(73) Patentinhaber : **BOEHRINGER MANNHEIM GMBH**
**Sandhofer Strasse 116**
**D-6800 Mannheim 31 (DE)**

(72) Erfinder : **von der Saal, Wolfgang, Dr. rer. nat.**
**Neuer Burgweg 3**
**D-6940 Weinheim (DE)**
Erfinder : **Friebe, Walter-Gunar, Dr. rer. nat.**
**Sophienstrasse 8**
**D-6800 Mannheim 1 (DE)**
Erfinder : **Mertens, Alfred, Dr. rer. nat.**
**Beethovenstrasse 20**
**D-6905 Schriesheim (DE)**
Erfinder : **Müller-Beckmann, Bernd, Dr. med. vet.**
**Hochgewanne 46**
**D-6718 Grünstadt (DE)**
Erfinder : **Sponer, Gisbert, Dr. med. vet.**
**Lessingstrasse 13**
**D-6941 Laudenbach (DE)**

EP 0 257 334 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue tricyclische Benzotriazole der allgemeinen Formel I

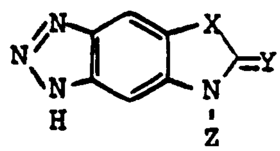 (I),

in welcher
Z Wasserstoff oder eine $C_1$-$C_3$-Acylgruppe darstellt,
Y Sauerstoff, Schwefel oder $H_2$ bedeutet und
X Sauerstoff, Schwefel, die Gruppe

$$>CR_1R_2$$

oder die Gruppe

$$>NR_3$$

darstellt, wobei
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_3$-Alkyliden- oder $C_3$-$C_7$-Cycloalkylidengruppe bilden,
$R_3$ entweder Wasserstoff oder eine $C_1$-$C_6$- Alkylgruppe bedeutet, mit Ausnahme der Verbindung 5-Acetyl-6,7-dihydrotriazolo[4,5f]indol deren Tautomere und deren physiologisch vertraegliche Salze anorganischer und organischer Saeuren und Verfahren zu ihrer Herstellung, sowie diese Verbindungen enthaltende Arzneimittel.

Die Verbindung 5-Acetyl-6,7-dihydrotriazolo[4,5f]indol ist in Chem. Abstr. 73, 709 741 a beschrieben, jedoch ohne Angabe eine Verwendung als Arzneimittel.

Da die Verbindungen der allgemeinen Formel I fuer den Fall, daß $R_1$ nicht gleich $R_2$ ist, ein asymmetrisches Kohlenstoffatom besitzen, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbesseren die Mikrozirkulation.

Für den Fall, daß X in der allgemeinen Formel I die Gruppe

$$>CR_1R_2$$

darstellt, können die Substituenten $R_1$ und $R_2$ gleich oder verschieden sein und $R_1$ Wasserstoff, eine Alkyl-, Cycloalkyl-oder Alkenylgruppe, und $R_2$ Wasserstoff, eine Alkyl-, Alkenyl- oder Cyangruppe oder eine durch eine Hydroxy-, Alkyl-, Amino-, Alkylamino-, Dialkylamino- oder Hydrazinogruppe substituierte Carbonylgruppe darstellen, wobei jeder der vorgenannten Alkyl- und Alkenylteile geradkettig oder verzweigt sein kann und 1-6 bzw. 2-6 Kohlenstoffatome und der vorgenannte Cycloalkylteil 3-7 Kohlenstoffatome enthalten kann.

Bevorzugt in diesem Sinne sind fuer $R_1$ bzw. $R_2$ Wasserstoff, die Methyl-, Ethyl-, Isopropyl-, 3-Pentyl-, Allyl-, Cyclopentyl-, Cyclohexyl-, Cyan-, Acetyl-, Propionyl-, Methoxycarbonyl-, Ethoxycarbonyl-, Aminocarbonyl-, Methylaminocarbonyl-, Dimethylaminocarbonyl- und Hydrazinocarbonylgruppe.

$R_1$ und $R_2$ koennen zusammen mit dem C-Atom, an das sie gebunden sind, auch einen Cycloalkylring mit 3-7 Kohlenstoffatomen bilden, vorzugsweise handelt es sich dabei um die Spirocyclopropyl-, Spirocyclobutyl-, Spirocyclopentyl- und Spirocyclohexylgruppe.

$R_1$ und $R_2$ koennen zusammen auch eine $C_1$-$C_6$ Alkyliden- oder $C_3$-$C_7$ Cycloalkylidengruppe bilden, vorzugsweise handelt es sich dabei um die Isopropylidengruppe.

Bedeutet in der allgemeinen Formel I X die Gruppe

$$>NR_3,$$

so sind unter $R_3$ Wasserstoff sowie lineare und verzweigte Alkylketten mit 1-6 C-Atomen zu verstehen, bevorzugt Methyl-, Ethyl-, Propyl- und Butylreste.

Bedeutet in der allgemeinen Formel I Z eine Acylgruppe, so sind darunter lineare aliphatische Acylgruppen mit 1-3 C-Atomen, vorzugsweise die Acetylgruppe zu verstehen.

Besonders bevorzugte Verbindungen sind Verbindungen der allgemeinen Formel I, in der
Z ein Wasserstoffatom oder eine Acetylgruppe darstellt,
Y Sauerstoff, Schwefel oder $H_2$ bedeutet,
X Sauerstoff, Schwefel oder die Gruppe

$$>CR_1R_2$$

darstellt, wobei
$R_1$ und $R_2$ gleich sind und die Methyl- oder Ethylgruppe darstellen,
$R_1$ und $R_2$ verschieden sind und $R_1$ Wasserstoff, die Methyl-, Ethyl-, Isopropyl-, Cyclopentylgruppe und $R_2$ eine Cyan-, Acetyl-, Methoxycarbonyl-, Ethoxycarbonyl-, Aminocarbonyl- und Hydrazinocarbonylgruppe bedeuten,
$R_1$ und $R_2$ einen Spirocyclopentylring darstellen, wenn $R_1$ und $R_2$ mit dem C-Atom, an das sie gebunden sind, einen Cycloalkylring bilden, oder
X die Gruppe $NR_3$ darstellt, wobei $R_3$ Wasserstoff, die Methyl- oder die Ethylgruppe bedeutet.

Die Verbindungen der allgemeinen Formel I koennen nach literaturbekannten Verfahren hergestellt werden. Besonders vorteilhaft sind jedoch die in Schema 1-3 gezeigten Synthesewege.

Schema 1

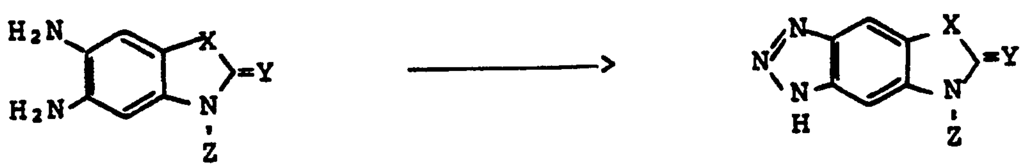

(II) (I)

Nach literaturbekannten Verfahren (vgl. F.R. Benson und W.L. Savell, Chemical Reviews 46, 1 (1950)) setzt man die ortho-Phenylendiaminderivate der allgemeinen Formel II, in der X, Y und Z die oben genannten Bedeutungen besitzen, mit salpetriger Saeure, dargestellt aus Alkalinitriten, wie z.B. Natriumnitrit, in Saeuren wie z.B. Essigsaeure, Salzsaeure oder Schwefelsaeure, um und erhaelt dabei die Verbindungen der allgemeinen Formel I, in der X, Y und Z die oben genannten Bedeutungen besitzen. Die Reaktionen fuehrt man bei −20°C bis +10°C durch, wobei man gegen Ende der Reaktion zur Vervollstaendigung auf 60°C-80°C erhitzt.

Zur Uebertragung des dritten Stickstoffatoms auf Verbindungen der allgemeinen Formel II eignen sich auch Diazoniumsalze, wie z.B. diazotierte Sulfanilsaeure.

Die Verbindungen der allgemeinen Formel II sind literaturbekannt oder lassen sich nach bekannten Verfahren herstellen (vgl. EP-OS 161 632 sowie deutsche Patentanmeldung P 35 24 067.9).

Schema 2

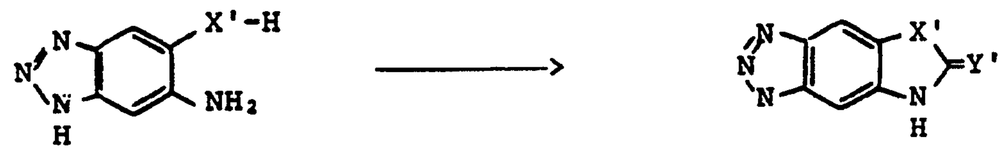

(III) (I')

Verbindungen der allgemeinen Formel I', in der X' Sauerstoff, Schwefel oder die Gruppe

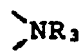

bedeutet und Y' Sauerstoff oder Schwefel darstellt, sind aus Verbindungen der allgemeinen Formel III zugaenglich, in der X' die oben genannten Bedeutungen besitzt. Dazu setzt man mit Reagenzien um, die Carbonylgruppen oder Thiocarbonylgruppen uebertragen, wie z.B. Phosgen, Thiophosgen, N,N'-Carbonyldiimidazol oder Harnstoff (vgl. E.S. Schipper und A.R. Day, in R.C. Elderfield (Ed.), Heterocyclic Compounds, Vol. 5, J. Wiley and Sons, New York 1957, S. 284 ; J.W. Cornforth, ebenda S. 439 ; J.M. Sprague und A.H. Land, ebenda S. 548).

Die Reaktionen mit Phosgen oder Thiophosgen fuehrt man dabei vorzugsweise in Salzsaeure bei Raumtemperatur, die Reaktionen mit N,N'-Carbonyldiimidazol in siedendem Dioxan durch.

Schema 3

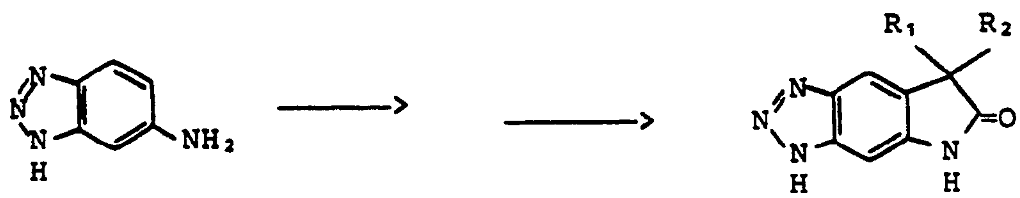

(IV) (I")

Alternativ zu Schema 1 lassen sich Verbindungen der allgemeinen Formel I" auch nach dem in Schema 3 vorgestellten Weg der Oxindol-Synthese herstellen (vgl. P.L. Julian, E.W. Meyer und H.C. Printy, in R.C. Elderfield (Ed.), Heterocyclic Compounds, Vol. 3, John Wiley and Sons, New York 1952, S. 128-142), z.B. durch

Hinsberg-Synthese : Umsetzung aromatischer Amine mit den Bisulfit-Additions-Verbindungen von Ketonen.

Brunner-Synthese : Cyclisierung aromatischer Amine ueber das Hydrazid zu Oxindolen.

Stollé-Synthese : Cyclisierung aromatischer Amine ueber ein Amid zum Oxindol.

Verbindungen der allgemeinen Formel I koennen auch nachtraeglich in eine andere Verbindung der allgemeinen Formel I umgewandelt werden. Dies trifft zum Beispiel zu

a) fuer die Umwandlung von Verbindungen der allgemeinen Formel I, in der X die angegebene Bedeutung hat, Z Wasserstoff und Y ein Sauerstoffatom darstellt, in Verbindungen der Formel I, in der Y ein Schwefelatom darstellt.

Die Umsetzung wird nach literaturbekannten Verfahren mit einem das Schwefelatom uebertragenden Reagenz, wie z.B. Phosphorpentasulfid durchgefuehrt, wobei man zweckmaeßigerweise 1-5 Mol $P_2S_5$ vorzugsweise jedoch 1 Mol in einem geeigneten Loesungsmittel umsetzt. Als Loesungsmittel kommen Tetrahydrofuran, Dioxan, Benzol, Toluol oder Pyridin bei Temperaturen zwischen 25 und 125°C in

Betracht.

Bevorzugt ist jedoch Pyridin bei einer Reaktionsdauer von 1-10 Stunden, vorzugweise jedoch 5 Stunden in Abhaengigkeit vom Reaktionspartner.

b) fuer die Umwandlung von Verbindungen der allgemeinen Formel I, in der X und Y die angegebenen Bedeutungen besitzen und Z eine Acylgruppe darstellt, in eine Verbindung der allgemeinen Formel I, in der Z ein Wasserstoffatom bedeutet.

Diese Hydrolysen fuehrt man zweckmaeßigerweise saeure- oder basenkatalysiert in einem geeigneten Loesungsmittel wie Wasser oder waessrigem Ethanol durch. Als Saeuren verwendet man etwa Salzsaeure, Schwefelsaeure oder Phosphorsaeure, als Basen Natronlauge oder Triethylamin.

c) fuer die Umwandlung von Verbindungen der allgemeinen Formel I, in der Y und Z die angegebenen Bedeutung haben und X die Gruppe

$$>CR_1R_2$$

darstellt, wobei $R_1$ die angegebenen Bedeutungen hat und $R_2$ eine Carboxylgruppe darstellt, in eine Verbindung der allgemeinen Formel I, in der $R_2$ eine durch eine Alkoxy-, Amino-, Alkylamino-, Dialkylamino- oder Hydrazinogruppe substituierte Carbonylgruppe darstellt.

Dies geschieht zweckmaeßigerweise dadurch, daß man ein reaktionsfaehiges Derivat der Carbonsaeure, wie z.B. ein Saeurechlorid, mit Alkoholen, primaeren oder sekundaeren Aminen in einem Loesungsmittel wie Methylenchlorid, Ether, Toluol oder Dimethylformamid gegebenenfalls in Gegenwart einer Base wie Triethylamin oder Pyridin bei Temperaturen zwischen −10°C und der Siedetemperatur des Loesungsmittels durchfuehrt. Ebenso gelingt die Umwandlung von Carbonsaeureestern zu Amiden oder Hydraziden, vorzugsweise in einem Loesungsmittel wie Ethanol in Gegenwart von 2-6 Mol eines primaeren oder sekundaeren Amins oder von Hydrazinhydrat bei Temperaturen zwischen 20°C und 100°C.

d) fuer die Umwandlung von Verbindungen der allgemeinen Formel I, in der Y und Z die angegebenen Bedeutungen haben und X die Gruppe

$$>CR_1R_2$$

darstellt, wobei $R_1$ die angegebenen Bedeutungen besitzt und $R_2$ eine Aminocarbonylgruppe darstellt, in eine Verbindungen der allgemeinen Formel I, in der $R_2$ eine Cyangruppe darstellt.

Die Umsetzung zur Cyangruppe wird zweckmaeßigerweise in einem inerten Loesungsmittel wie z.B. in Methylenchlorid, Chloroform, Dioxan, Pyridin, Xylol, Chlorbenzol in Gegenwart eines wasserentziehenden Mittels wie z.B. Thionylchlorid, Phosphoroxytrichlorid, Phosphorpentoxid, Phosphorpentachlorid, Aluminiumchlorid, Benzolsulfonsaeurechlorid, Toluolsulfonsaeurechlorid, Triphenylphosphin, Bortrifluorid oder Polyphosphorsaeureethylester bei Temperaturen zwischen 50 und 250°C vorzugsweise jedoch bei der Siedetemperatur des Loesungsmittels durchgefuehrt.

e) fuer die Umwandlung von Verbindungen der allgemeinen Formel I, in der Y und Z die angegebenen Bedeutungen besitzen und X die Gruppe

$$>CR_1R_2$$

darstellt, wobei $R_1$ die angegebenen Bedeutungen besitzt und $R_2$ eine Cyan- oder Alkoxygruppe darstellt, in eine Verbindung der allgemeinen Formel I, in der $R_2$ eine Carboxylgruppe oder ein Wasserstoffatom darstellt.

Diese Hydrolysen fuehrt man zweckmaeßigerweise entweder in Gegenwart einer Saeure wie Salzsaeure, Schwefelsaeure, Phosphorsaeure oder Trichloressigsaeure oder in Gegenwart einer Base wie

Natriumhydroxid oder Kaliumhydroxid in einem geeigneten Loesungsmittel wie Wasser, Wasser/Methanol, Ethanol, Wasser/Ethanol, Wasser/Isopropanol oder Wasser/Dioxan bei Temperaturen zwischen –10°C und 120°C, z.B. bei Temperaturen zwischen Raumtemperatur und der Siedetemperatur des Reaktionsgemisches durch.

An diese Verseifung schließt sich eine spontane Decarboxylierung an, falls man bei erhoehter Temperatur, z.B. beim Siedepunkt des Loesungsmittels oder Loesungsmittelgemisches arbeitet.
f) fuer die Umwandlung von Verbindungen der allgemeinen Formel I in denen $R_1$ = $R_2$ = Wasserstoff bedeuten zu Verbindungen der allgemeinen Formel I in denen $R_1$ zusammen mit $R_2$ die Isopropylidengruppe, die Cyclopentyliden- oder Cyclohexylidengruppe darstellen, sowie gegebenenfalls deren Hydrierung zu den entsprechenden Verbindungen der allgemeinen Formel I, in denen $R_1$ oder $R_2$ gleich Wasserstoff ist.
Dies betrifft z.B. die Umsetzung mit Verbindungen der allgemeinen Formel V

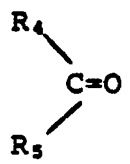

(V),

in welcher $R_4$ und $R_5$ Alkylgruppen sind oder $R_4$ und $R_5$ zusammen eine $C_3$-$C_7$-Cycloalkylengruppe bilden, in Gegenwart einer Base wie Ammoniak oder Triethylamin in alkoholischer Loesung.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen sie die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure), Gelatine, Agar-Agar, Calciumphosphat, Magnesiumstearat, tierische und pflanzlische Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 1-500 mg pro Tag bezogen auf 75 kg Koerpergewicht appliziert. Bevorzugt ist es, 2-3 mal pro Tag 1-2 Tabletten mit einem Wirkstoffgehalt von 1-200 mg zu verabreichen. Die Tabletten koennen auch retardiert sein, wodurch nur noch 1 mal pro Tag 1-2 Tabletten mit 1-500 mg Wirkstoff gegeben werden muß. Der Wirkstoff kann auch durch Injektion 1-8 mal pro Tag bzw. durch Dauerinfusion gegeben werden, wobei Mengen von 1-500 mg/Tag normalerweise ausreichen.

Bevorzugt im Sinne der vorliegenden Erfindung sind außer den in den Beispielen genannten Verbindungen die folgenden und deren Tautomere :
6,7-Dihydro-1H,5H-pyrrolo[2,3-f]benzotriazol-6-on
7-Ethyl-6,7-dihydro-1H,5H-pyrrolo[2,3-f]benzotriazol-6-on

7-n-Propyl-6,7-diyhdro-1H,5H-pyrrolo[2,3-f]benzotriazol-6-on
7-i-Propyl-6,7-dihydro-1H,5H-pyrrolo[2,3-f]benzotriazol-6-on
7,7-Diethyl-6,7-dihydro-1H,5H-pyrrolo[2,3-f]benzotriazol-6-on
7-Methyl-7-ethoxycarbonyl-6,7-dihydro-1H,5H-pyrrolo[2,3-f]benzatriazol-6-on
7-Ethoxycarbonyl-6,7-dihydro-1H,5H-pyrrolo[2,3-f]benzotriazol-6-on
7-Methyl-7-aminocarbonyl-6,7-dihydro-1H,5H-pyrrolo[2,3-f]benzatriazol-6-on
7-Aminocarbonyl-6,7-dihydro-1H,5H-pyrrolo[2,3-f]benzotriazol-6-on
7-Methyl-7-hydrazinocarbonyl-6,7-dihydro-1H,5H-pyrrolo[2,3-f]benzotriazol-6-on
7-Hydrazinocarbonyl-6,7-dihydro-1H,5H-pyrrolo[2,3-f]benzotriazol-6-on
7-Methyl-7-acetyl-6,7-dihydro-1H,5H-pyrrolo[2,3-f]benzotriazol-6-on
7-Methyl-7-cyano-6,7-dihydro-1H,5H-pyrrolo[2,3-f]benzotriazol-6-on
7-Cyano-6,7-dihydro-1H,5H-pyrrolo[2,3-f]benzotriazol-6-on
Spiro[cyclohexan-1,7′-6′,7′-dihydro-3′H,5′H-pyrrolo[2′,3′-f]-benztriazol]-6′-on
Spiro[cyclopropan-1,7′-6′,7′-dihydro-3′H,5′H-pyrrolo[2′,3′-f]triazol]-6′-on
7-Ethyl-1,5,6,7-tetrahydroimidazo[4,5-f]benzotriazol-6-on
7-Propyl-1,5,6,7-tetrahydroimidazo[4,5-f]benzotriazol-6-on
7,7-Dimethyl-6,7-dihydro-1H,5H-pyrrolo[2,3-f]benzotriazol-6-thion
7-Methyl-1,5,6,7-tetrahydroimidazo[4,5-f]benzotriazol-6-thion
5,6-Dihydro-1H-oxazolo[4,5-f]benzotriazol-6-thion
5,6-Dihydro-1H-thiazolo[4,5-f]benzotriazol-6-thion
7-Ethoxycarbonyl-7-methyl-6,7-dihydro-1H,5H-pyrrolo[2,3-f]benzatriazol-6-on

Beispiel 1

7,7-Dimethyl-6,7-dihydro-1H,5H-pyrrolo[2,3-f]benzotriazol-6-on

Zu einer Suspension von 570 mg (3 mMol) 5,6-Diamino-3,3-dimethyl-indolin-2-on in 6 ml 1 N Salzsaeure gab man portionsweise 240 mg (3.5 mMol) $NaNO_2$, wobei man die Temperatur durch Eiskuehlung zwischen 0°C und +5°C hielt. Es entstand eine rote Loesung, die man fuenf Minuten auf 50°C bis 60°C erhitzte. Nach Abkuehlen auf Raumtemperatur saugte man den kristallinen Niederschlag ab und wusch mit Wasser. Man erhielt 380 mg der Titelverbindung als rote Kristalle mit dem Schmp. 288-291°C (Zers.).

Beispiel 2

7-Methyl-6,7-dihydro-1H,5H-pyrrolo[2,3-f]benzotriazol-6-on

mit dem Schmp. >300°C erhielt man in 12% Ausbeute analog dem Beispiel 1 aus 5,6-Diamino-3-methyl-indolin-2-on nach säulenchromatographischer Reinigung (Kieselgel, Dichlormethan, Methanol 95 :5) und Umkristallisation aus Isopropanol.

Beispiel 3

Spiro[cyclopentan-1,7′-6′, 7′-dihydro-1′H,5′H-pyrrolo[2′,3′-f]-benzatriazol]-6′-on

mit dem Schmp. 271-272°C (Zers.) erhielt man in 34% Ausbeute analog dem Beispiel 1 aus 5′-6′-Diamino-spiro[cyclopentan-1,3′-indolin]-2′-on nach säulenchromatographischer Reinigung und Umkristallisation aus Essigester.

Beispiel 4

5,6-Dihydro-1H-oxazolo[4,5-f]benzotriazol-6-on

erhielt man analog dem Beispiel 1 in 16% Ausbeute aus 5,6-Diaminobenzoxazolin-2-on. Nach säulenchromatographischer Reinigung (Kieselgel 60, Dichlormethan :Methanol :Eisessig = 10 :1 :0.2) und Verrühren mit Essigester hat die Substanz einen Schmp. >360°C.

Beispiel 5

7-Methyl-1,5,6,7-tetrahydroimidazo[4,5-f]benzotriazol-6-on

mit dem Schmp. >300°C erhielt man in 23% Ausbeute analog dem Beispiel 1 aus 5,6-Diamino-1-methylbenzimidazolin-2-on nach säulenchromatographischer Reinigung (Kieselgel, Dichlormethan :Methylenchlorid = 95 :5) und Umkristallisation aus Methanol.

Beispiel 6

5,6-Dihydro-1H-thiazolo[4,5-f]benzotriazol-6-on

mit dem Schmp. 355-360°C (Zers.) erhielt man in 13% Ausbeute analog dem Beispiel 1 aus 5,6-Diaminobenzothiazolin-2-on nach saeulenchromatographischer Reinigung (Kieselgel, Butanol :Essigsaeure :Wasser = 4 :1 :5, untere Phase verworfen).

Beispiel 7

5-Acetyl-7,7-dimethyl-6,7-dihydro-1H,5H-pyrrolo[2,3-f]benzatriazol

Eine Loesung aus 2.0 g (9.1 mmol) 1-Acetyl-5,6-diamino-3,3-dimethyl-indolin-dihydrochlorid, 6 ml Wasser und 1.8 ml Essigsaeure wird auf 0°C gekuehlt und mit einer Loesung von 0.62 g (9.1 mmol) Natriumnitrit in 1 ml Wasser versetzt. Man ruehrt 1 h bei Raumtemperatur, verduennt mit 5 ml Wasser und filtriert. Es verbleiben 1.4 g (67% d.Th.) Titelverbindung vom Schmp. 281-283°C.

Beispiel 8

7,7-Dimethyl-6,7-dihydro-1H,5H-pyrrolo[2,3-f]benzotriazol-dihydrochlorid

0.5 g der vorstehend beschriebenen Verbindung werden mit 20 ml ethanolischer Chlorwasserstoffloesung 2.5 h zum Rueckfluß erhitzt. Man laeßt abkuehlen und filtriert. Man isoliert 0.4 g (70% d.Th.) der Titelverbindung vom Schmp. 243-245°C.

## Ansprüche

1. Tricyclische Benzotriazole der allgemeinen Formel I

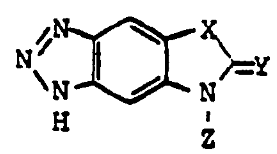

(I),

in welcher
Z Wasserstoff oder eine $C_1$-$C_3$ Acylgruppe darstellt,
Y Sauerstoff, Schwefel oder $H_2$ bedeutet und
X Sauerstoff, Schwefel, die Gruppe

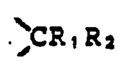

oder die Gruppe

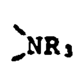

darstellt, wobei

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet, $R_2$ ein Wasserstoffatom, eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden.

$R_3$ entweder Wasserstoff oder eine $C_1$-$C_6$-Alkylgruppe bedeutet, mit Ausnahme der Verbindung 5-Acetyl-6,7-dihydrotriazolo[4,5f]indol deren Tautomere und deren physiologisch vertraegliche Salze anorganischer und organischer Saeuren.

2. Tricyclische Benzotriazole der allgemeinen Formel I gemäß Anspruch 1, in welcher

Z ein Wasserstoffatom oder eine Acetylgruppe darstellt,

Y Sauerstoff, Schwefel oder $H_2$ bedeutet,

X Sauerstoff, Schwefel oder die Gruppe

$$>CR_1R_2$$

darstellt, wobei

$R_1$ und $R_2$ gleich sind und die Methyl- oder Ethylgruppe darstellen,

$R_1$ und $R_2$ verschieden sind und $R_1$ Wasserstoff, die Methyl-, Ethyl-, Isopropyl-, Cyclopentylgruppe und $R_2$ eine Cyan-, Acetyl-, Methoxycarbonyl-, Ethoxycarbonyl-, Aminocarbonyl- und Hydrazinocarbonylgruppe bedeuten,

$R_1$ und $R_2$ einen Spirocyclopentylring darstellen, wenn $R_1$ und $R_2$ mit dem C-Atom, an das sie gebunden sind, einen Cycloalkylring bilden, oder

X die Gruppe

$$>NR_3$$

darstellt, wobei $R_3$ Wasserstoff, die Methyl- oder die Ethylgruppe bedeutet,

deren Tautomere und deren physiologisch verträgliche Salze anorganischer und organischer Säuren.

3. Tricyclische Benzotriazole der allgemeinen Formel I gemäß Anspruch 1 oder 2, in der

Z ein Wasserstoffatom oder eine Acetylgruppe darstellt,

Y Sauerstoff oder $H_2$ bedeutet,

X Sauerstoff, Schwefel, die Gruppe

$$>CR_1R_2$$

oder die Gruppe

$$>NR_3$$

darstellt, wobei

$R_1$ und $R_2$ ein Wasserstoffatom oder eine Methylgruppe bedeutet, oder $R_1$ und $R_2$ zusammen mit dem C-Atom, an das sie gebunden sind, einen Spirocyclopentylgring bilden, und

$R_3$ ein Wasserstoffatom oder eine Methylgruppe bedeuten, mit Ausnahme der Verbindung 5-Acetyl-6,7-dihydrotriazolo[4,5f]indol,

deren Tautomere und deren physiologisch verträgliche Salze anorganischer und organischer Säuren.

4. Verfahren zur Herstellung von tricyclischen Benzotriazolen der allgemeinen Formel I

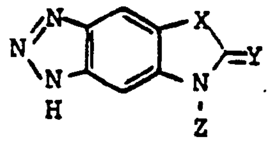

(I),

in welcher
Z Wasserstoff oder eine $C_1$-$C_3$-Acylgruppe darstellt,
Y Sauerstoff, Schwefel oder $H_2$ bedeutet und
X Sauerstoff, Schwefel, die Gruppe

$>CR_1R_2$

oder die Gruppe

$>NR_3$

darstellt, wobei
$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,
$R_2$ ein Wasserstoffatom, eine $C_1$-$C_6$-Alkyl-, $C_2$-$C_6$-Alkenyl- oder Cyangruppe, eine durch eine Hydroxy-, $C_1$-$C_6$-Alkyl-, $C_1$-$C_6$-Alkoxy-, Amino-, $C_1$-$C_6$-Alkylamino-, Di-$C_1$-$C_6$-alkylamino- oder Hydrazinogruppe substituierte Carbonylgruppe oder mit $R_1$ zusammen eine $C_3$-$C_7$-Cycloalkylengruppe darstellt oder $R_1$ und $R_2$ zusammen eine $C_1$-$C_6$-Alkyliden- oder $C_3$-$C_7$-Cycloalkylidengruppe bilden,
$R_3$ entweder Wasserstoff oder eine $C_1$-$C_6$-Alkylgruppe bedeutet, mit Ausnahme der Verbindung 5-Acetyl-6,7-dihydrotriazolo[4,5f]indol deren Tautomere und deren physiologisch vertraegliche Salze anorganischer und organischer Saeuren,

dadurch gekennzeichnet, daß man in an sich bekannter Weise
a) eine Verbindung der Formel II

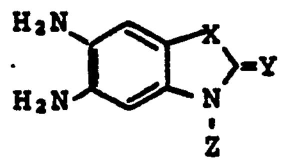

(II)

in der X, Y und Z die angegebene Bedeutung haben, mit salpetriger Säure oder einem Diazoniumsalz umsetzt, oder
b) für den Fall, daß X in der Formel I Sauerstoff, Schwefel oder die Gruppe

$>NR_3$

bedeuten und Y Sauerstoff oder Schwefel darstellen,
eine Verbindung der Formel III

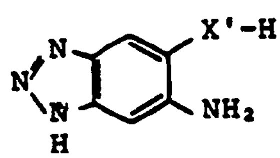

(III)

in der X′ Sauerstoff, Schwefel oder die Gruppe

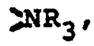

wobei $R_3$ die angegebene Bedeutung hat, bedeuten, mit Phosgen, Thiophosgen, N,N'-Dicarbonyldiimidazol oder Harnstoff umsetzt, oder

c) für den Fall, daß X die Gruppe

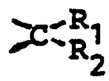

und Y Sauerstoff bedeutet,

eine Verbindung der Formel IV

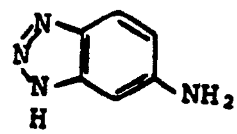

(IV)

mit Bisulfat-Additions-Verbindungen von entsprechenden Ketonen (Hinsberg-Synthese) oder über ein entsprechendes Hydrazid (Brunner-Synthese) oder über ein entsprechendes Amid (Hollé-Synthese) cyclisiert

und anschließend gewünschtenfalls erhaltene Verbindungen der Formel I in andere Verbindungen der Formel I umwandelt sowie gegebenenfalls die Verbindungen in pharmakologisch verträgliche Salze überführt.

5. Verfahren zur Herstellung von tricyclischen Benzotriazolen der allgemeinen Formel I gemäß Anspruch 4,

in der

Z ein Wasserstoffatom oder eine Acetylgruppe darstellt,

Y Sauerstoff, Schwefel oder $H_2$ bedeutet,

X Sauerstoff, Schwefel oder die Gruppe $CR_1R_2$ darstellt, wobei

$R_1$ und $R_2$ gleich sind und die Methyl- oder Ethylgruppe darstellen,

$R_1$ und $R_2$ verschieden sind und $R_1$ Wasserstoff, die Methyl-, Ethyl-, Isopropyl-, Cyclopentylgruppe und $R_2$ eine Cyan-, Acetyl-, Methoxycarbonyl-, Ethoxycarbonyl-, Aminocarbonyl- und Hydrazinocarbonylgruppe bedeuten,

$R_1$ und $R_2$ einen Spirocyclopentylring darstellen, wenn $R_1$ und $R_2$ mit dem C-Atom, an das sie gebunden sind, einen Cycloalkylring bilden, oder

X die Gruppe $NR_3$ darstellt, wobei $R_3$ Wasserstoff, die Methyl- oder die Ethylgruppe bedeutet.

6. Verfahren zur Herstellung von tricyclischen Benzotriazolen der allgemeinen Formel I gemäß Anspruch 4 oder 5,

in der

Z ein Wasserstoffatom oder eine Acetylgruppe darstellt,

Y Sauerstoff oder $H_2$ bedeutet,

X Sauerstoff, Schwefel, die Gruppe

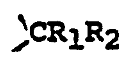

oder die Gruppe

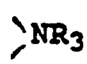

darstellt, wobei

$R_1$ und $R_2$ ein Wasserstoffatom oder eine Methylgruppe bedeutet, oder $R_1$ und $R_2$ zusammen mit dem C-Atom, an das sie gebunden sind, einen Spirocyclopentylring bilden, und

$R_3$ ein Wasserstoffatom oder eine Methylgruppe bedeuten, mit Ausnahme der Verbindung 5-Acetyl-6,7-dihydrotriazolo[4,5f]indol, deren Tautomere und deren physiologisch verträgliche Salze anorganischer und organischer Säuren.

7. Arzneimittel, enthaltend mindestens eine Verbindung gemäß Anspruch 1, 2 oder 3.

8. Verbindungen gemäß Anspruch 1, 2 oder 3 zur Verwendung als Wirkstoffe für Arzneimittel zur Behandlung von Herz- und Kreislauferkrankungen.

9. Verwendung von Verbindungen gemäß Anspruch 1, 2 oder 3 zur Herstellung von Arzneimitteln zur Behandlung von Herz- und Kreislauferkrankungen.

## Claims

1. Tricyclic benzotriazoles of the general formula I

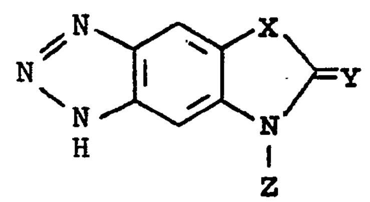

(I),

in which Z represents hydrogen or a $C_1$-$C_3$-acyl group, Y signifies oxygen, sulphur or $H_2$, X represents oxygen, sulphur, the group

$$> CR_1R_2$$

or the group

$$> NR_3,$$

whereby $R_1$ signifies a hydrogen atom, a $C_1$-$C_6$-alkyl, $C_2$-$C_6$-alkenyl or a $C_6$-$C_7$-cycloalkyl group, $R_2$ signifies a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or, together with $R_1$, represents a $C_3$-$C_7$-cycloalkylene group or $R_1$ and $R_2$ together form a $C_1$-$C_6$-alkylidene or $C_3$-$C_7$-cycloalkylidene group, $R_3$ signifies either hydrogen or a $C_1$-$C_6$-alkyl group, with the exception of the compound 5-acetyl-6,7-dihydrotriazolo[4,5-f]indole, their tautomers and their physiologically acceptable salts of inorganic and organic acids.

2. Tricyclic benzotriazoles of the general formula I according to claim 1, in which Z represents a hydrogen atom or an acetyl group, Y signifies oxygen, sulphur or $H_2$, X represents oxygen, sulphur or the group

$$> CR_1R_2,$$

whereby $R_1$ and $R_2$ are the same and represent the methyl or ethyl group, $R_1$ and $R_2$ are different and $R_1$ signifies hydrogen, the methyl, ethyl, isopropyl, cyclopentyl group and $R_2$ a cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$ form a cycloalkyl ring with the C-atom to which they are attached or X represents the group

$$> NR_3,$$

whereby $R_3$ signifies hydrogen, the methyl or the ethyl group, their tautomers and their physiologically acceptable salts of inorganic and organic acids.

3. Tricyclic benzotriazoles of the general formula I according to claim 1 or 2, in which Z represents a hydrogen atom or an acetyl group, Y signifies oxygen or $H_2$, X represents oxygen, sulphur or the group

$> CR_1R_2$

or the group

$> NR_3$,

whereby $R_1$ and $R_2$ signify a hydrogen atom or a methyl group or $R_1$ and $R_2$, together with the C-atom to which they are attached, form a spirocyclopentyl ring and $R_3$ signifies a hydrogen atom or a methyl group, with the exception of the compound 5-acetyl-6,7-dihydrotriazolo[4,5-f]indole, their tautomers and their physiologically acceptable salts of inorganic and organic acids.

4. Process for the preparation of tricyclic benzotriazoles of the general formula I

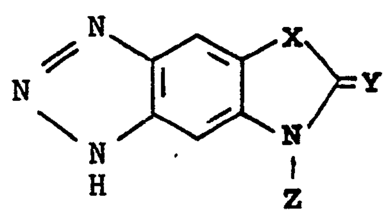

(I)

in which Z represents hydrogen or a $C_1$-$C_3$-acyl group, Y signifies oxygen, sulphur or $H_2$ and X represents oxygen, sulphur, the group

$> CR_1R_2$

or the group

$> NR_3$,

whereby $R_1$ signifies a hydrogen atom, a $C_1$-$C_6$-alkenyl or a $C_3$-$C_7$-cycloalkyl group, $R_2$ represents a hydrogen atom, a $C_1$-$C_6$-alkyl, $C_2$-$C_6$-alkenyl or cyano group, a carbonyl group substituted by a hydroxyl, $C_1$-$C_6$-alkyl, $C_1$-$C_6$-alkoxy, amino, $C_1$-$C_6$-alkylamino, di-$C_1$-$C_6$-alkylamino or hydrazino group or, together with $R_1$, represents a $C_3$-$C_7$-cycloalkylene group or $R_1$ and $R_2$ together form a $C_1$-$C_6$-alkylidene or $C_3$-$C_7$-cycloalkylidene group, $R_3$ signifies either hydrogen or a $C_1$-$C_6$-alkyl group, with the exception of the compound 5-acetyl-6,7-dihydrotriazole[4,5-f]indole, of their tautomers and of their physiologically acceptable salts of inorganic and organic acids, characterised in that, in per se known manner, one

a) reacts a compound of the formula II

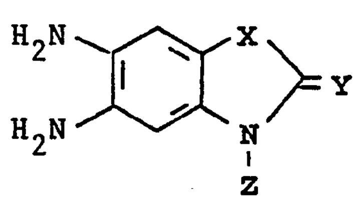

(II),

in which X, Y and Z have the given meaning, with nitrous acid or a diazonium salt ; or for the case that X in the formula I signifies oxygen, sulphur or the group

$> NR_3$

and Y represents oxygen or sulphur, reacts a compound of the formula III

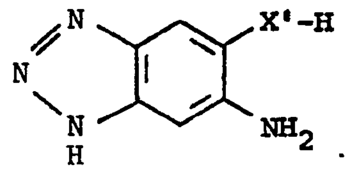

(III)

in which X' signifies oxygen, sulphur or the group

$>NR_3$,

whereby $R_3$ has the given meaning, with phosgene, thiophosgene, N,N'-dicarbonyldiimidazole or urea; or

c) for the case that X signifies the group

$>CR_1R_2$

and Y oxygen atom, cyclises a compound of the formula IV

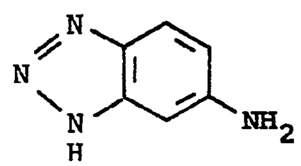

(IV)

with bisulphite addition compounds of corresponding ketones (Hinsberg synthesis) or via a corresponding hydrazide (Brunner synthesis) or via a corresponding amide (Stollé synthesis) ; and subsequently, if desired, converts compounds obtained of the formula I into other compounds of the formula I, as well as possibly converts the compounds into pharmacologically acceptable salts.

5. Process for the preparation of tricyclic benzotriazoles of the general formula I according to claim 4, in which Z represents a hydrogen atom or an acetyl group, Y signifies oxygen, sulphur atom or $H_2$, X represents oxygen, sulphur or the group

$>CR_1R_2$,

whereby $R_1$ and $R_2$ are the same and represent the methyl or ethyl group, $R_1$ and $R_2$ are different and $R_1$ signifies hydrogen, the methyl, ethyl, isopropyl, cyclopentyl group and $R_2$ a cyano, acetyl, methoxycarbonyl, ethoxycarbonyl, aminocarbonyl and hydrazinocarbonyl group, $R_1$ and $R_2$ represent a spirocyclopentyl ring when $R_1$ and $R_2$, with the C-atom to which they are attached, form a cycloalkyl ring or X represents the

$>NR_3$,

whereby $R_3$ signifies hydrogen, the methyl or the ethyl group.

6. Process for the preparation of tricyclic benzotriazoles of the general formula I according to claim 4 or 5, in which Z represents a hydrogen atom or an acetyl group, Y signifies oxygen or $H_2$, X represents oxygen, sulphur or the group

$>CR_1R_2$

or the group

$>NR_3$,

whereby $R_1$ and $R_2$ signify a hydrogen atom or methyl group or $R_1$ and $R_2$, together with the C-atom to which they are attached, form a spirocyclopentyl ring and $R_3$ signifies a hydrogen atom or a methyl group, with the exception of the compound 5-acetyl-6,7-dihydrotriazolo[4,5-f]indole, of their tautomers and of their physiologically acceptable salts of inorganic and organic acids.

7. Medicaments containing at least one compound according to claim 1, 2 or 3.

8. Compounds according to claim 1, 2 or 3 for use as active materials for medicaments for the treatment of heart and circulatory diseases.

9. Use of compounds according to claim 1, 2 or 3 for the preparation of medicaments for the treatment of heart and circulatory diseases.

**Revendications**

1. Benzotriazoles tricycliques de formule générale I

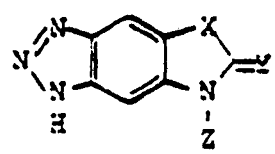

(I),

où
Z représente un atome d'hydrogène ou un groupe acyle en $C_1$-$C_3$,
Y représente un atome d'oxygène, de soufre ou de $H_2$, et
X représente un atome d'oxygène, de soufre, le groupe

$>CR_1R_2$

ou le groupe

$>NR_3$,

dans lesquels
$R_1$ représente un atome d'hydrogène, un groupe alkyle en $C_1$-$C_6$, alcényle en $C_2$-$C_6$ ou un groupe cycloalkyle en $C_3$-$C_7$,
$R_2$ représente un atome d'hydrogène, un groupe alkyle en $C_1$-$C_6$, alcényle en $C_2$-$C_6$ ou cyano, un groupe carbonyle substitué par un groupe hydroxy, un groupe alkyle en $C_1$-$C_6$, alcoxy en $C_1$-$C_6$, amino, alkyl($C_1$-$C_6$)amino, dialkyl($C_1$-$C_6$)amino ou hydrazino, ou forme, conjointement avec $R_1$, un groupe cycloalkylène en $C_3$-$C_7$ ou $R_1$ et $R_2$ forment, conjointement, un groupe alkylidène en $C_1$-$C_6$ ou cycloalkylidène en $C_3$-$C_7$,
$R_3$ représente soit un atome d'hydrogène soit un groupe alkyle en $C_1$-$C_6$, à l'exception du composé 5-acétyl-6,7-dihydrotriazolo[4,5 f]-indol, leurs tautomères et leurs sels physiologiquement acceptables, formés avec des acides minéraux ou organiques.

2. Benzotriazoles tricycliques de formule générale I selon la revendication 1, où
Z représente un atome d'hydrogène ou un groupe acétyle,
Y représente un atome d'oxygène, de soufre ou de $H_2$,
X représente un atome d'oxygène, de soufre ou le groupe

$>CR_1R_2$,

$R_1$ et $R_2$ sont identiques et représentent le groupe méthyle ou éthyle,
$R_1$ et $R_2$ sont différents et $R_1$ représente un atome d'hydrogène, le groupe méthyle, éthyle, isopropyle, cyclo-

pentyle et $R_2$ représente un groupe cyano, acétyle, méthoxycarbonyle, éthoxycarbonyle, aminocarbonyle et hydrazinocarbonyle,

$R_1$ et $R_2$ représentent un cycle spirocyclopentyle, lorsque $R_1$ et $R_2$ forment, conjointement avec l'atome de C auquel ils sont liés, un cycle cycloalkyle, ou

X représente le groupe

$$>NR_3,$$

où $R_3$ représente un atome d'hydrogène, le groupe méthyle ou éthyle,

leurs tautomères et leurs sels physiologiquement acceptables, formés avec des acides minéraux et organiques.

3. Benzotriazoles tricycliques de formule générale I selon la revendication 1 ou 2, où

Z représente un atome d'hydrogène ou un groupe acétyle,

Y représente un atome d'oxygène ou de $H_2$,

X représente un atome d'oxygène, de soufre, le groupe

$$>CR_1R_2$$

ou le groupe

$$>NR_3,$$

$R_1$ et $R_2$ représentent un atome d'hydrogène ou un groupe méthyle, ou $R_1$ et $R_2$ forment, conjointement avec l'atome de C auquel ils sont liés, un cycle spirocyclopentyle, et

$R_3$ représente un atome d'hydrogène ou un groupe méthyle, à l'exception du composé 5-acétyl-6,7-dihydro-triazolo[4,5 f]-indol, leurs tautomères et leurs sels physiologiquement acceptables, formés avec des acides minéraux et organiques.

4. Procédé de préparation de benzotriazoles tricycliques de formule générale I

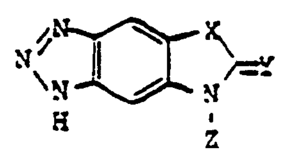

(I),

où

Z représente un atome d'hydrogène ou un groupe acyle en $C_1$-$C_3$,

Y représente un atome d'oxygène, de soufre ou de $H_2$, et

X représente un atome d'oxygène, de soufre, le groupe

$$>CR_1R_2$$

ou le groupe

$$>NR_3,$$

dans lesquels

$R_1$ représente un atome d'hydrogène, un groupe alkyle en $C_1$-$C_6$, alcényle en $C_2$-$C_6$ ou un groupe cycloalkyle en $C_3$-$C_7$, représente un atome d'hydrogène, un groupe alkyle en $C_1$-$C_6$, alcényle en $C_2$-$C_6$ou cyano, un groupe carbonyle substitué par un groupe hydroxy, un groupe alkyle en $C_1$-$C_6$, alcoxy en $C_1$-$C_6$, amino, alkyl($C_1$-$C_6$)amino, dialkyl($C_1$-$C_6$)amino ou hydrazino, ou forme, conjointement avec $R_1$, un groupe cycloalkylène en $C_3$-$C_7$ ou $R_1$ et $R_2$ forment, conjointement, un groupe alkylidène en $C_1$-$C_6$ ou cycloalkylidène en $C_3$-$C_7$,

$R_3$ représente soit un atome d'hydrogène soit un groupe alkyle en $C_1$-$C_6$, à l'exception du composé 5-acétyl-6,7-dihydrotriazolo[4,5f]-indol, leurs tautomères et leurs sels physiologiquement acceptables, formés avec des acides minéraux ou organiques, caractérisé en ce que, de manière connue en soi,

a) on fait réagir un composé de formule II

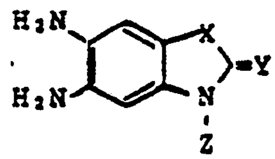

(II)

où X, Y, Z ont les significations mentionnées, avec l'acide nitrique ou avec un sel de diazonium, ou

b) dans le cas où X dans la formule I représente un atome d'oxygène, de soufre ou le groupe

$$>NR_3,$$

et Y représente un atome d'oxygène ou de soufre,
on fait réagir un composé de formule III

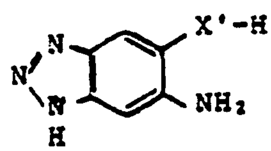

(III)

où X′ représente un atome d'oxygène, de soufre ou le groupe

$$>NR_3,$$

$R_3$ ayant la signification mentionnée, avec le phosgène, le thiophosgène, le N,N′-dicarbonyldiimidazole ou l'urée, ou

c) dans le cas où X représente le groupe

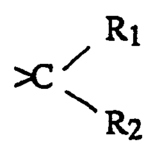

et Y représente un atome d'oxygène,
on cyclise un composé de formule IV

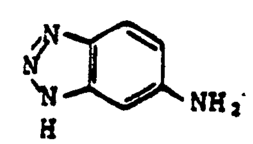

(IV)

avec des composés d'addition de bisulfate de cétones appropriées (synthèse de Hirnsberg) ou par l'intermédiaire d'un hydrazide approprié (synthèse de Brunner) ou par l'intermédiaire d'un amide approprié (synthèse de Hollé) et ensuite, on transforme éventuellement les composés de formule I obtenus en

d'autres composés de formule I, et éventuellement, on transforme ces composés en leurs sels pharmacologiquement acceptables.

5. Procédé de préparation de benzotriazoles tricycliques de formule générale I, selon la revendication 4, où

Z représente un atome d'hydrogène ou un groupe acétyle,
Y représente un atome d'oxygène, de soufre ou de $H_2$,
X représente un atome d'oxygène, de soufre ou le groupe

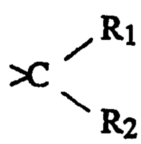

$R_1$ et $R_2$ sont identiques et représentent le groupe méthyle ou éthyle,
$R_1$ et $R_2$ sont différents et $R_1$ représente un atome d'hydrogène, le groupe méthyle, éthyle, isopropyle, cyclopentyle et $R_2$ représente un groupe cyano, acétyle, méthoxycarbonyle, éthoxycarbonyle, aminocarbonyle et hydrazinocarbonyle,
$R_1$ et $R_2$ représentent un cycle spirocyclopentyle, lorsque $R_1$ et $R_2$ forment, conjointement avec l'atome de C auquel ils sont liés, un cycle cycloalkyle ou
X représente le groupe

$$>NR_3,$$

où $R_3$ représente un atome d'hydrogène, le groupe méthyle ou éthyle.

6. Procédé de préparation de benzotriazoles tricycliques de formule générale I, selon la revendication 4 ou 5,
où
Z représente un atome d'hydrogène ou un groupe acétyle,
Y représente un atome d'oxygène ou de $H_2$,
X représente un atome d'oxygène, de soufre, le groupe

$$>CR_1R_2$$

ou le groupe

$$>NR_3,$$

$R_1$ et $R_2$ représentent un atome d'hydrogène ou un groupe méthyle, ou $R_1$ et $R_2$ forment, conjointement avec l'atome de C auquel ils sont liés, un cycle spirocyclopentyle,et
$R_3$ représente un atome d'hydrogène ou un groupe méthyle, à l'exception du composé 5-acétyl-6,7-dihydro-triazolo[4,5f]-indol, leurs tautomères et leurs sels physiologiquement acceptables, formés avec des acides minéraux et organiques.

7. Médicament, contenant au moins un composé selon la revendication 1, 2 ou 3.

8. Composés selon la revendication 1, 2 ou 3, pour l'utilisation comme composant actif dans des médicaments pour le traitement de maladies cardiovasculaires.

9. Utilisation des composés selon la revendication 1, 2 ou 3, pour la préparation de médicaments pour le traitement de maladies cardiovasculaires.